# EUROPÄISCHE PATENTSCHRIFT

(11) **EP 1 622 873 B1**
(45) Veröffentlichungstag und Bekanntmachung des Hinweises auf die Patenterteilung: **13.01.2010**
(21) Anmeldenummer: 04728780.0
(22) Anmeldetag: 22.04.2004
(51) Int. Cl.: C07D 215/20

(54) **VERFAHREN ZUR HERSTELLUNG VON RAC-1-{4-[2-HYDROXY-3-(5-QUINOLYLOXY)PROPYL]PIPERAZIN-1-YL}-2,2-DIPHENYLETHAN-1-ON FUMARAT**
METHOD FOR PRODUCING RAC-1-{4-[2-HYDROXY-3-(5-QUINOLYLOXY)PROPYL]-PIPERAZIN-1-YL}-2,2-DIPHENYLETHAN-1-ONE FUMARATE
PROCEDE DE PRODUCTION DE FUMARATE DE RAC-1-{4-[2-HYDROXY-3-(5-QUINOLYLOXY)PROPYL]-PIPERAZINE-1-YLE} 2,2-DIPHENYLETHANE-1-ONE

(30) Priorität: 06.05.2003 DE 10321255
(43) Veröffentlichungstag der Anmeldung: 08.02.2006
(73) Patentinhaber: Bayer Schering Pharma Aktiengesellschaft, 13353 Berlin (DE)
(72) Erfinder: WEINMANN, Hilmar, 16548 Glienicke (DE); SCHNEIDER, Matthias, 10589 Berlin (DE); GOTTFRIED, Michael, 10439 Berlin (DE)
(86) Internationale Anmeldenummer: PCT/EP2004/004277
(87) Internationale Veröffentlichungsnummer: WO 2004/099151

(56) Entgegenhaltungen:
- EP-A- 0 363 212
- EP-A- 0 575 890
- PATENT ABSTRACTS OF JAPAN Bd. 2000, Nr. 13, 5. Februar 2001 (2001-02-05) -& JP 2000 281653 A (MITSUI CHEMICALS INC), 10. Oktober 2000 (2000-10-10) in der Anmeldung erwähnt
- TSUNEJI SUZUKI ET AL: "+Structure-Activity Relationship of Newly Synthesized Quinoline Derivatives for Reversal of Multidrug Resistance in Cancer" JOURNAL OF MEDICINAL CHEMISTRY, AMERICAN CHEMICAL SOCIETY. WASHINGTON, US, Bd. 40, Nr. 13, 1997, Seiten 2047-2052, XP000926067 ISSN: 0022-2623 in der Anmeldung erwähnt

## Beschreibung

Die Erfindung betrifft ein Verfahren zur Herstellung von reinstem rac-1-{4-[2-Hydroxy-3-(5-quinolyloxy)propyl]-piperazin-1-yl}-2,2-diphenylethan-1-one fumarat

Der Multi Drug Resistance Modulator rac-1-{4-[2-Hydroxy-3-(5-quinolyloxy)propyl]-piperazin-1-yl}-2,2-diphenylethan-1-one fumarate, dessen Herstellung und Verwendung als karzinostatisches Arzneimittel wird neben weiteren Derivaten dieser Verbindung in der EP 575890 beschrieben.

Nach dem in der EP 575890 beschriebenen Verfahren zur Herstellung von reinem rac-1-{4-[2-Hydroxy-3-(5-quinoiyloxy)propyl]-piperazin-1-yl}-2,2-diphenylethan-1-one fumarate wird zunächst durch Kopplung der beiden Bausteine Epoxilin (B) (5-(2,3-Epoxypropoxy)-quinolin) und Diphenpiperazid (C) (N-(2,2-Diphenylacetyl)piperazin) die freie Base 5-[3-{4-(2,2-Diphenylacetyl)piperazin-1-yl}-2-hydroxypropoxy]quinolin als Rohprodukt isoliert. Diese Umsetzung beinhaltet zwei Teilstufen. Zuerst wird das Epoxylat mit Hydroxychinolin (A) umgesetzt. Im zweiten Schritt wird das Epoxilin (B) (5-(2,3-Epoxypropoxy)-quinolin) durch Diphenpiperazid (C) (N-(2,2-Diphenylacetyl)piperazin) geöffnet, es entsteht der sekundäre Alkohol (D). Diese Reaktion findet in Ethanol statt, Wasser katalysiert die Umsetzung. Die Aufarbeitung / Isolierung erfolgt dann durch Fällung aus Aceton/Wasser und Trocknung im Vakuum bei 60 °C.

Die Gesamtreaktion ergibt sich aus dem nachfolgenden Schema:

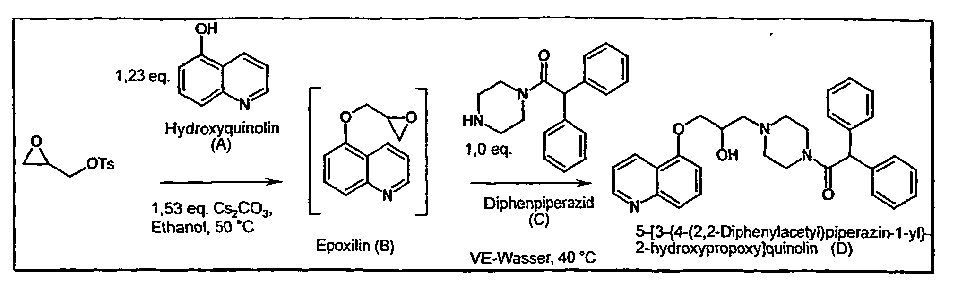

An die Isolierung der freien Base, die noch viele Verunreinigungen enthält (Reinheit des Rohproduktes typischerweise ca. 80 %), schließt sich im nächsten Schritt ein sehr aufwendiges Reinigungsverfahren an. Nach Aktivkohlebehandlung der freien Base und der Bildung des Fumarats in Methanol wird zur Aufreinigung erneut die freie Base durch Behandlung mit verdünnter Natronlauge hergestellt. Anschließend erfolgt als letzter Schritt die wiederholte Fumarat-Bildung. Die beiden Fumarat-Bildungen sind verfahrenstechnisch identisch und unterscheiden sich nur durch die Ansatzgröße (T. Suzuki et al., J. Med. Chem. (1997) 40, 2047)(JP 2000281653). Ausgehend von dem Rohprodukt freie Base beträgt die typische Laborausbeute für diese Reinigungssequenz 45 % der Theorie.

Nachteilig an diesem Verfahren ist dabei nicht nur die geringe Ausbeute (über 50 % Verlust auf der Endstufe), sondern auch die aufwendige technische Durchführung, die viele betriebliche Kapazitäten bindet und damit erhöhte Kosten verursacht. Besonders nachteilig ist dabei die extrem schlechte Filtrierbarkeit der freien Base, die teilweise über mehrere Wochen filtergetrocknet werden muss.

Trotz des hohen verfahrenstechnischen Aufwandes können nach diesem bekannten Verfahren die extrem hohen Reinheitsanforderungen an rac-1-{4-[2-Hydroxy-3-(5-quinolyloxy)propyl]piperazin-1-yl}-2,2-diphenytethan-1-one fumarat nicht immer völlig zufriedenstellend erreicht werden.

Ferner liefert das in der EP 575 890 beschriebenen Verfahren keine vernünftigen Ergebnisse beim Scale up.

Eine Übersicht über die einzelnen Reaktionen gibt das folgende Schema:

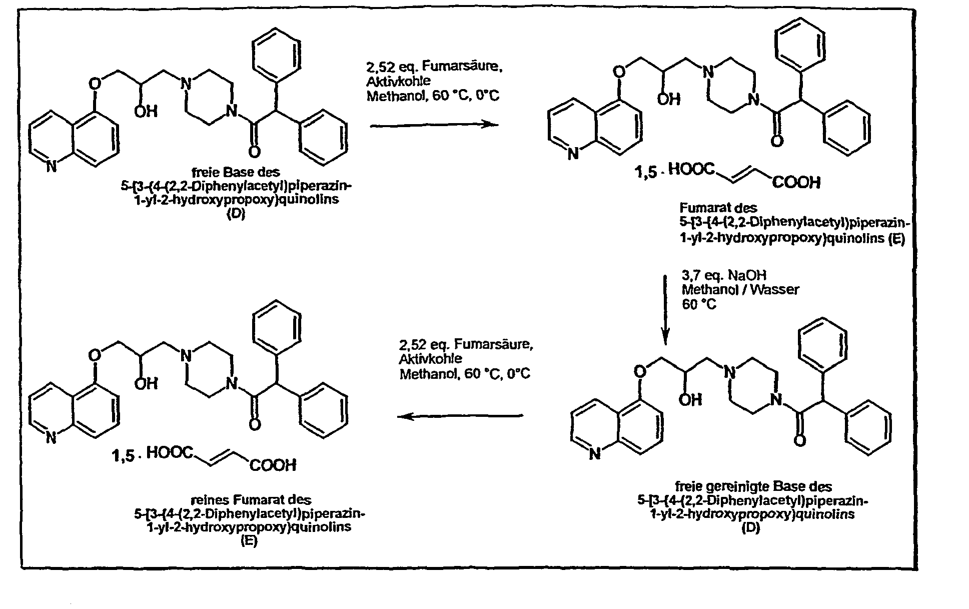

Es wurde nun gefunden, dass mit dem erfindungsgemässen Verfahren diese bekannten Nachteile überwunden werden können. Bei dem erfindungsgemässen Verfahren wird zunächst ebenfalls das Epoxilin (B) und Diphenpiperazid (C) durch Öffnung des Epoxids gekoppelt. Anschließend wird jedoch nicht die freie Base (D) sondern nach Zugabe von fester Fumarsäure direkt das Fumaratsalz (E) als Rohprodukt isoliert.

Gegenstand der Anmeldung ist somit ein Verfahren zur Herstellung von reinstem rac-1-{4-[2-Hydroxy-3-(5-quinolyloxy)propyl]-piperazin-1-yl}-2,2-diphenylethan-1-one fumarat, das dadurch gekennzeichnet ist, dass man zunächst
a) ein Epoxytosylat der Struktur I
   mit 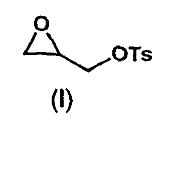
b) 5-Hydroxychinolin (II) 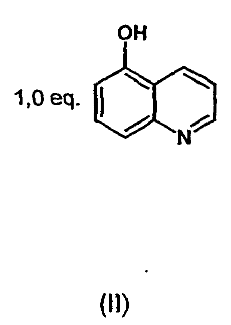 und Cäsiumcarbonat in einem geeigneten Lösungsmittel und bei geeigneter Temperatur zum 5-(2,3-Epoxypropoxy)-quinolin der Formel III 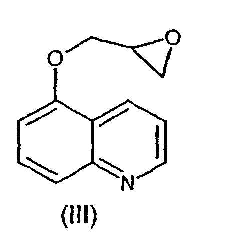 reagieren lässt, und anschliessend das 5-(2,3-Epoxypropoxy)-quinolin der Formel III
c) mit N-(2,2-Diphenylacetyl)piperazin der Formel IV 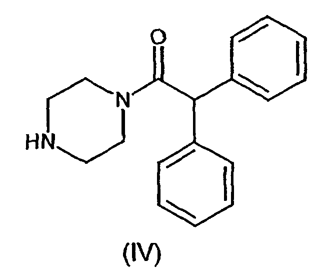 in einem geeigneten Lösungsmittel und bei geeigneter Temperatur unter anschliessender
   Zugabe fester Fumarsäure zum rohen rac-1-{4-[2-Hydroxy-3-(5-quinolyloxy)propyl]-piperazin-1-yl}-2,2-diphenylethan-1-on fumarat der Formel V 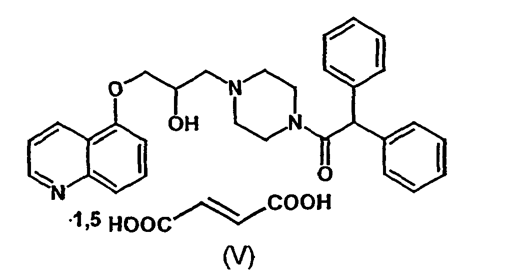 umsetzt und anschliessend
d) das so gebildete rohe rac-1-{4-[2-Hydroxy-3-(5-quinolyloxy)propyl]-piperazin-1-yl}-2,2-diphenylethan-1-on fumarat (V) isoliert und in einem Lösungsmittelgemisch aus Methanol und Methylenchlorid gelöst wird, mit Aktivkohle behandelt wird und anschliessend über einen Druckfilter mit Kieselgel als Säulenmaterial filtriert wird, und das so gewonnene reine rac-1-{4-[2-Hydroxy-3-(5-quinolyloxy)propyl]-piperazin-1-yl}-2,2-diphenylethan-1-on fumarat (V) aus einem geeigneten Alkohol kristallisiert wird.

Die Umsetzung erfolgt vorzugsweise in einem Temperaturbereich von 35 bis 60°C.

Geeignete Lösungsmittel sind z. B. Ketone, wie Aceton, Methyl-isobutylketon etc., Alkohole, wie Methanol, Ethanol, Isopropanol etc., Amide, wie Dimethylformamid, Dimethylacetamid, N-Methyl-pyrrolidon etc.

Im Vergleich zu den bekannten Verfahren weist das über das erfindungsgemässe Verfahren isolierte rohe rac-1-{4-[2-Hydroxy-3-(5-quinolyloxy)propyl]-piperazin-1-yl}-2,2-diphenylethan-1-on der Verfahrenstufe c) bereits eine dramatisch gesteigerte Reinheit von 90 - 96 % auf.

Der in Verfahrensstufe d) enthaltene Reinigungsschritt wird vorzugsweise in einem Lösungsmittelgemisch aus MeOH/MeCl₂ im Verhältnis 3:7, v:v durchgeführt.

Als Aktivkohle kommt vorzugsweise Norit SX Plus zur Anwendung.

Vorzugsweise beträgt die Kieselgel-Menge die 3fache Menge bezogen auf das Ausgangsmaterial.

Vorzugsweise beträgt die Temperatur bei der Kristallisation in Verfahrensstufe (d) 0 °C_

Die typische Laborausbeute für den Aufreinigungsschritt (d) beträgt 84 % der Theorie.

### Herstellungsbeispiel

### Herstellung von rac-1-{4-[2-Hydroxy-3-(5-quinolyloxy)propyl]-piperazin-1-yl}-2,2-diphenylethan-1-on fumarat

A) Unter Stickstoff werden 44,2 g 5-Hydroxy-chinolin und 151,9 g Cäsiumcarbonat mit 560 ml Aceton bei Raumtemperatur zusammen geben und für 30 Minuten bei 60 °C Badtemperatur gerührt.
   Bei 50 °C Innentemperatur werden 73,0 g Epoxytosylat, gelöst in
   153,3 g Dichlormethan, zugeben. Es wird zwei Stunden bei 50 °C nachgerührt. Der Ansatz wird bei 50 °C filtriert. Der Filferrückstand (anorganische Salze) wird mit 560 ml auf 50 °C angewärmtem Aceton nachgewaschen. Anschliessend werden 85,4 g N-(2,2-Diphenylacetyl)piperazin zugeben und einer Badtemperatur von 40 °C im Vakuum auf 374 g Endgewicht eingeengt. Es werden anschliessend 374 g VE-Wasser zugeben und 2 Stunden bei 40 °C gerührt. Danach werden 255 g Aceton und 201 g VE-Wasser zugeben. Der Ansatz wird auf Raumtemperatur abgekühlt und es werden 89,1 g Fumarsäure in fester Form dazugege-ben. Es wird 60 Minuten bei 60 °C Badtemperatur gerührt und anschließend 2 Stunden bei 0 °C gerührt. Danach wird der Feststoff abgesaugt und mit 150 ml eiskaltem Methanol der Filterrückstand wird bei 60 °C im Vakuum getrocknet.
   Ausbeute: 65 - 85 % der Theorie
B) 56,0 g des so hergestellte rac-1-{4-[2-Hydroxy-3-(5-quinolyloxy)propyl]-piperazin-1-yl}-2,2-diphenylethan-1-on fumarasts wurden unter Stickstoff und bei Raumtemperatur mit 5,6 g Aktivkohle, Norit SX plus, 672 ml Methanol und 1008 ml Dichlormethan versetzt.
   Die entstehende Suspension wird bei einer Badtemperatur von 75°C auf Rückflußtemperatur erwärmen und 30 min am Rückfluß gehalten. Bei einer Innentemperatur von 40°C geht rac-1-{4-[2-Hydroxy-3-(5-quinolyloxy)propyl]-piperazin-1-yl}-2,2-diphenylethan-1-on fumarat in Lösung. Anschliessend wird der Ansatz heiß über 300% Kieselgel filtriert und das Kieselgel mit 560 ml einer Mischung aus 168 ml Methanol und 392 ml Dichlormethan bei Raumtemperatur RT nachgewaschen. Die Lösung wird bei einer Badtemperatur von 40°C und einem Anfangsvakuum von 400 mbar auf ein Endvolumen von 517 ml eingeengt. Das Endvakuum beträgt 350 mbar. Das abdestillierte Volumen entspricht etwa der Volumendifferenz (ca. 1,7 l). Es werden 404 ml Methanol zugegeben, so daß ein Endvolumen von 921 ml erreicht wird. Die Lösung wird auf 0°C gekühlt, wobei das Produkt ausfällt. Die entstehende Suspension wird 2 Stunden bei 0°C gerührt und anschliessend über ein Papierfilter filtriert. Der Filterrückstand wird mit 56,0 ml eiskaltem Methanol gewaschen. Der Filterrückstand wird bei 60 °C und im Vakuum bei 100 mbar 10 Stunden getrocknet.
   - Ausbeute (unkorr.):: 47,29 g ( 84,45 % v.E.)
   - Reinheit:: 99,65 % ( HPLC, 100 % Methode )

### Vergleich des literaturbekannten mit dem erfindungsgemässen Reinigungsverfahren

| | **Literaturbekanntes Reinigungsverfahren** | **Erfindungsgemässes Reinigungsverfahren** |
|---|---|---|
| **Gesamtausbeute der Synthese über alle Stufen** | 28 % der Theorie | 43 % der Theorie, |
| **Reinheit (HPLC, 100 % Methode)** | 98,54 %, mehrere VU über 0,1 % | 99,55 - 99,89 %, keine VU über 0,1 % |
| **Technischer Aufwand** | Hoch, mehrere Zwischenisolierungen sind erforderlich, ferner extrem schlechte Filtrierbarkeit | Deutlich reduzierter Zeitbedarf im Betrieb durch einfachere technische Durchführbarkeit. |

| | | |
|---|---|---|
| VU= Verunreinigung | | |

Aus den Ergebnissen ist ersichtlich, dass die Gesamtausbeute an rac-1-{4-[2-Hydroxy-3-(5-quinolyloxy)propyl]-piperazin-1-yl}-2,2-diphenylethan-1-on fumarat (V) um mehr als 50 % gesteigert werden kann.

Aus den Ergebnissen ist auch ersichtlich, dass mit dem erfindungsgemässen Verfahren die erforderlichen extrem hohen Reinheiten erreicht werden können, wobei der technische Aufwand gleichzeitig stark reduziert werden konnte. Ferner ist zu bemerken, dass die Verunreinigungen (VU)1 - 4, die während der Umsetzung gebildet werden, vollständig vermieden werden können.

Ein Vergleich von Chargen die nach dem bekannten Verfahren, als auch nach dem erfindungsgemässen Verfahren hergestellt worden sind, sind in der nachfolgenden Tabelle aufgeführt.

| **Verunreinigung (VU)** | **Bekanntes Verfahren** | **Erfindungsgemässes Verfahren** |
|---|---|---|
| 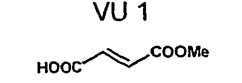 | 0.80 % | n. d. |
| 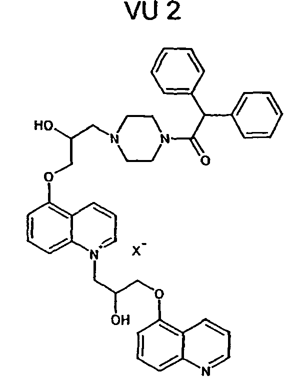 | 0.44 % | n. d. |
| 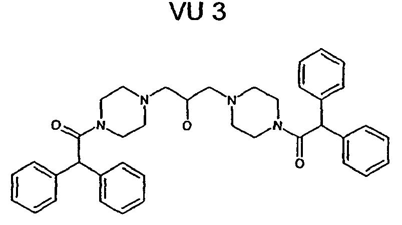 | 0.08 % | n. d. |
| 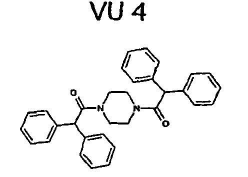 | 0.11% | n. d. |
| VU 5 Struktur nicht bekannt | < 0,1 % *) | 0,01 *) |
| 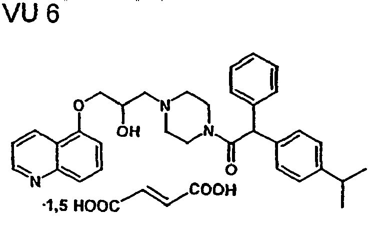 | < 0,1% *) | 0,02 *) |
| Reinheit | 98,54 % | 99,84 % |

| | | |
|---|---|---|
| n. d. = nicht detektierbar, da unter Detektionslimit *) abhängig von der Reinheit der Kaufware (Edukte): Falls im Edukt mit < 0,2 % enthalten. | | |

Weitere Verunreinigungen, die während der Synthese gebildet werden und ebenfalls durch das erfindungsgemässe Verfahren effektiv entfernt werden:

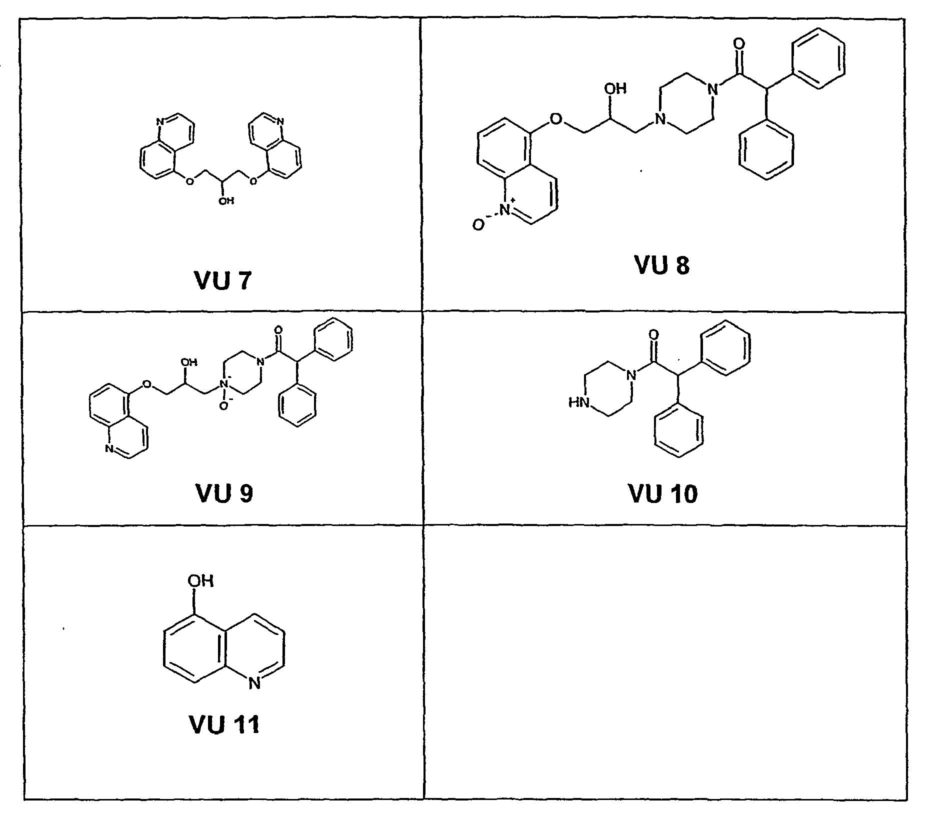

Das erfindungsgemässe Verfahren eignet sich insbesondere zur Herstellung von rac-1-{4-[2-Hydroxy-3-(5-quinolyloxy)propyl]-piperazin-1-yl}-2,2-diphenylethan-1-on fumarat in verfahrenstechnischer Grösse. So kann mit diesem Verfahren rac-1-{4-[2-Hydroxy-3-(5-quinolyloxy)propyl]-piperazin-1-yl}-2,2-diphenylethan-1-on fumarat erstmals in Mengen von mehr als 1000 g in der beschriebenen Reinheit hergestellt werden.

Gegenstand der vorliegenden Erfindung ist auch ein Verfahren, das als Verunreinigung weniger als 0,1 %, vorzugsweise weniger als 0,01 % an mindestens einer der Einzelverunreinigung (VU 1 - VU 11) umfasst.

Mit herkömmlichen Aufreinigungsverfahren, wie Chromatographie, Hochdruckflüssigkeitschromatographie (HPLC), Ionenaustauscher, etc., lässt sich rac-1-{4-[2-Hydroxy-3-(5-quinolyloxy)-propyl]-piperazin-1-yl}-2,2-diphenylethan-1-on fumarat nicht in der erfindungsgemässen Reinheit herstellen.

## Patentansprüche

1. Verfahren zur Herstellung von reinstem rac-1-{4-[2-Hydroxy-3-(5-quinolyloxy)propyl]-piperazin-1-yl}-2,2-diphenyletthan-1-one fumarat, **dadurch gekennzeichnet, dass** man zunächst
a) ein Epoxytosylat der Struktur I 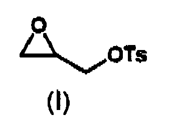 mit
b) 5-Hydroxychinolin (II) 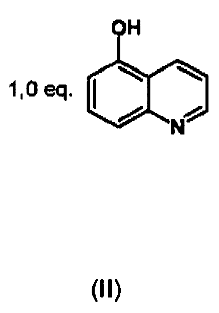 und Cäsiumcarbonat in einem geeigneten Lösungsmittel und bei geeigneter Temperatur zum 5-(2,3-Epoxypropoxy)-quinolin der Formel III 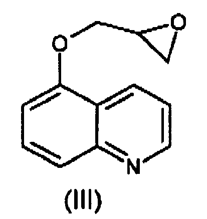 reagieren lässt, und anschliessend das 5-(2,3-Epoxypropoxy)-quinolin der Formel III
c) mit N-(2,2-Diphenylacetyl)piperazin der Formel IV 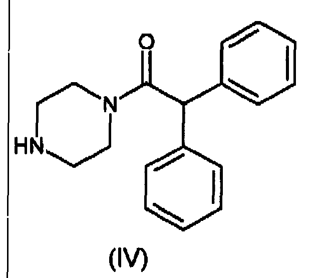 in einem geeigneten Lösungsmittel und bei geeigneter Temperatur unter anschliessender Zugabe fester Fumarsäure zum rohen rac-1-{4-[2-Hydroxy-3-(5-quinolyloxy)propyl]-piperazin-1-yl}-2,2-diphenylethan-1-on fumarat der Formel V 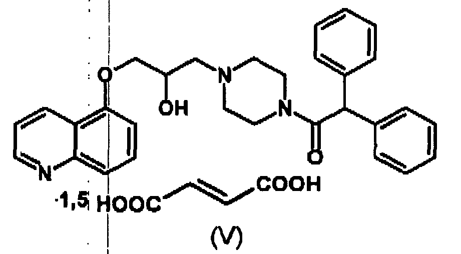 umsetzt und anschliessend
d) das so gebildete rohe rac-1-{4-[2-Hydroxy-3-(5-quinolyloxy)propyl]-piperazin-1-yl}-2,2-diphenylethan-1-on fumarat (V) isoliert und in einem Lösungsmittelgemisch aus Methanol und Methylenchlorid gelöst wird, mit Aktivkohle behandelt wird und anschliessend über Druckfilter mit Kieselgel als Säulenmaterial filtriert wird, und das so gewonnene reine rac-1-{4-[2-Hydroxy-3-(5-quinolyloxy)propyl]-piperazin-1-yl}-2,2-diphenylethan-1-on fumarat (V) aus einem geeigneten Alkohol kristallisiert wird.

2. Verfahren nach Anspruch 1, **dadurch gekennzeichnet, dass** als Lösungsmittel Aceton zur Anwendung kommt.

3. Verfahren nach Anspruch 1, **dadurch gekennzeichnet, dass** die Umsetzung in einem Temperaturbereich von 35 bis 60°C durchgeführt wird.

4. Verfahren nach Anspruch 1, **dadurch gekennzeichnet, dass** die Kieselgel-Menge die 3fache Menge bezogen auf das Ausgangsmaterial ist.

5. Verfahren nach Anspruch 1, **dadurch gekennzeichnet, dass** die Temperatur bei der Kristallisation in Verfahrensstufe (d) 0 °C beträgt.

6. Verfahren nach einer der Ansprüche 1 - 5, **dadurch gekennzeichnet, dass** ein rac-1-{4-[2-Hydroxy-3-(5-quinolyloxy)propyl]-piperazin-1-yl}-2,2-diphenylethan-1-on fumarat mit einer Reinheit von mehr als 98,54 % erhalten wird.

7. Verfahren nach einer der Ansprüche 1-5, **dadurch gekennzeichnet, dass** ein rac-1-{4-[2-Hydroxy-3-(5-quinolyloxy)propyl]piperazin-1-yl}-2,2-diphenylethan-1-on fumarat mit einer Reinheit von mindestens 98,55 % erhalten wird.

8. Verfahren nach einer der Ansprüche 1 - 5, **dadurch gekennzeichnet, dass** ein rac-1-{4-[2-Hydroxy-3-(5-quinolyloxy)propyl]-piperazin-1-yl}-2,2-diphenylethan-1-on fumarat mit einer Reinheit von mindestens 99,55 % erhalten wird.

9. Verfahren nach einer der Ansprüche 1-5, **dadurch gekennzeichnet, dass** ein rac-1-{4-[2-Hydroxy-3-(5-quinolyloxy)propyl]-piperazin-1-yl}-2,2-diphenylethan-1-on fumarat mit einer Reinheit von mindestens 99,65% erhalten wird.

10. Verfahren nach einer der Ansprüche 1 - 5, **dadurch gekennzeichnet, dass** ein rac-1-{4-[2-Hydroxy-3-(5-quinolyloxy)propyl]-piperazin-1-yl}-2,2-diphenylethan-1-on fumarat mit einer Reinheit von mindestens 99,84 % erhalten wird.

11. Verfahren nach einer der Ansprüche 1 - 5, **dadurch gekennzeichnet, dass** ein rac-1-{4-[2-Hydroxy-3-(5-quinolyloxy)propyl]-piperazin-1-yl}-2,2-diphenylethan-1-on fumarat gemäß den Ansprüchen 6 bis 10 in einer Menge von mehr als 1000 g erhalten wird.

12. Verfahren nach einer der Ansprüche 1 - 5, **dadurch gekennzeichnet, dass** ein rac-1-{4-[2-Hydroxy-3-(5-quinolyloxy)-propyl]-piperazin-1-yl}-2,2-diphenylethan-1-on fumarat gemäß den Ausbrüchen 6 bis 10, das als Verunreinigung weniger als 0,1% an mindestens einer der Einzelverunreinigung VU1-VU4 und VU6-VU11 umfasst, erhalten wird.

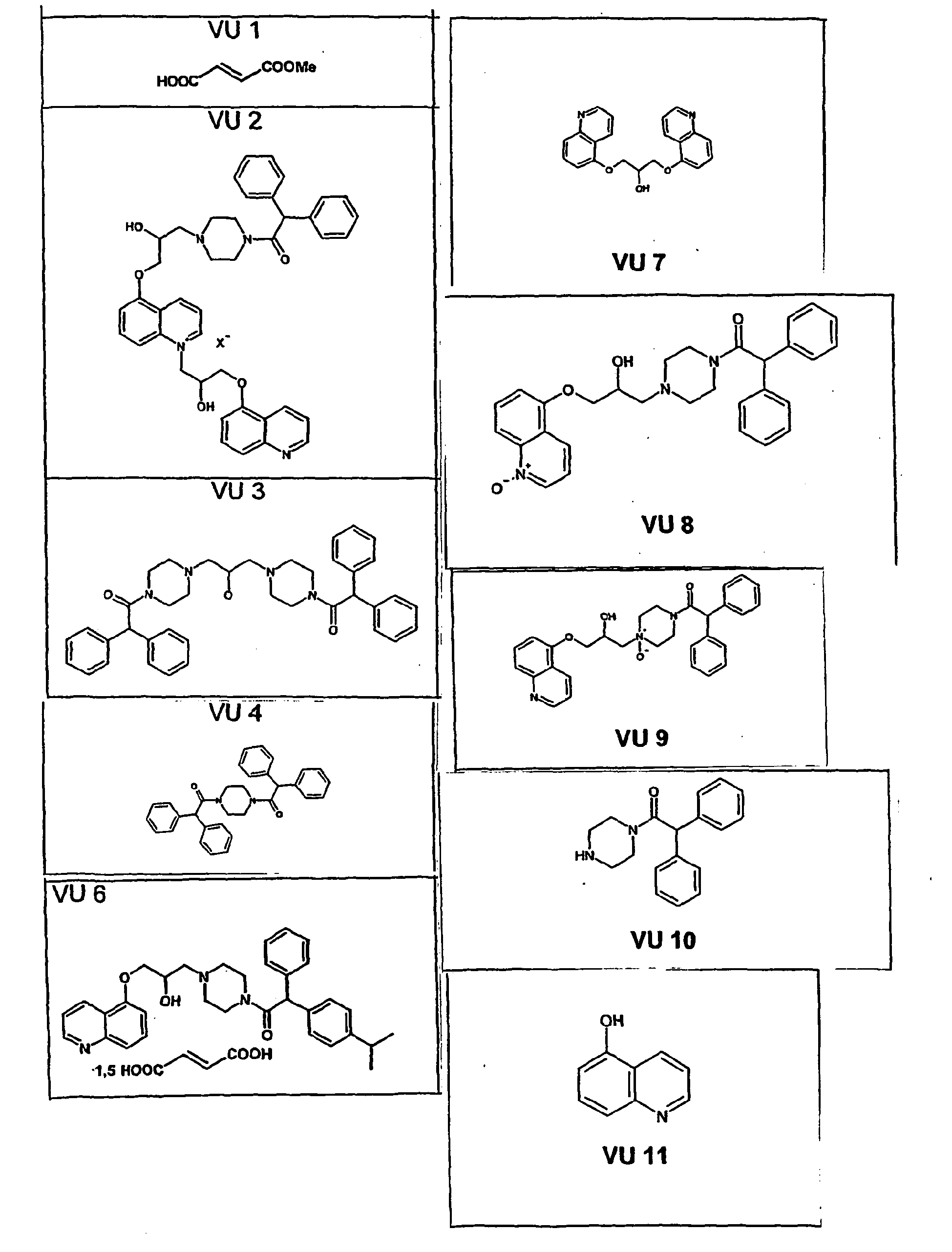

13. Verfahren nach einer der Ansprüche 1 - 5, **dadurch gekennzeichnet, dass** ein rac-1-{4-[2-Hydroxy-3-(5-quiholyloxy)-propyl]-piperazin-1-yl}-2,2-diphenylethan-1-on fumarat gemäß den Ansprüchen 6 bis 10, das als Verunreinigung weniger als 0,01 % an mindestens der Einzelverunreinigung VU1-VU4 und VU6-VU11 umfasst, erhalten wird.

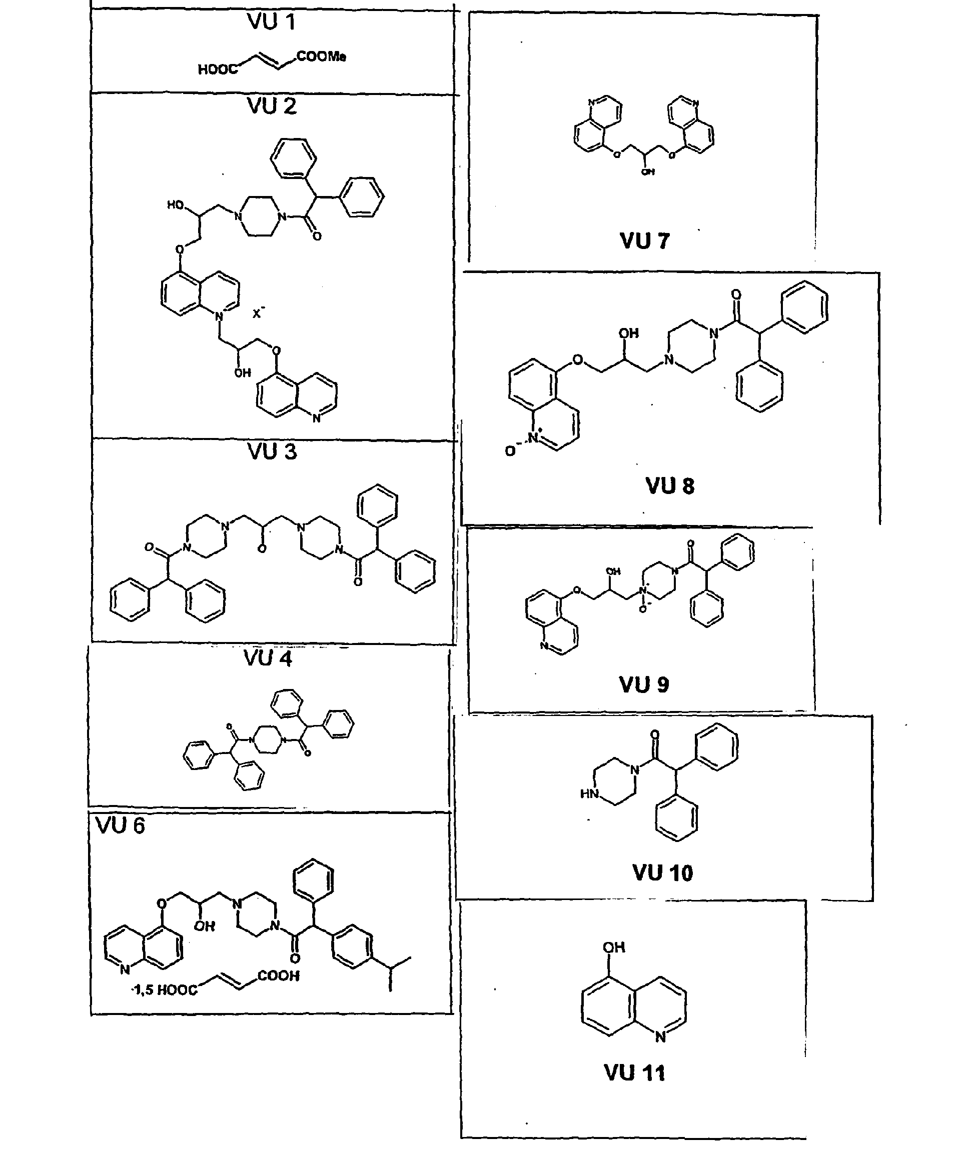

## Claims

1. Method for producing ultrapure rac-1-{4-[2-hydroxy-3-(5-quinolyloxy)propyl]piperazin-1-yl}-2,2-diphenylethan-1-one fumarate, **characterized in that** first
a) an epoxy tosylate of the structure I 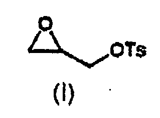 is reacted with
b) 5-hydroxyquinoline (II) 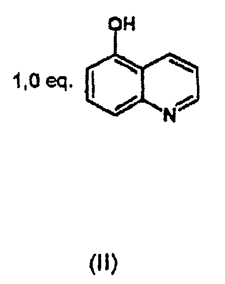 and caesium carbonate in a suitable solvent and at a suitable temperature to give the 5-(2,3-epoxypropoxy)quinoline of the formula III 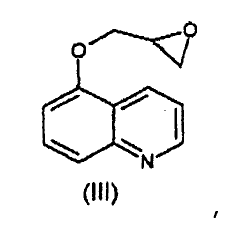 and subsequently the 5-(2,3-epoxypropoxy)-quinoline of the formula III
c) is reacted with N-(2,2-diphenylacetyl)-piperazine of the formula IV 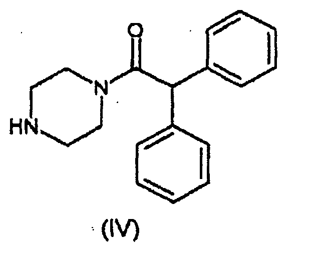 in a suitable solvent and at a suitable temperature with subsequent addition of solid fumaric acid to give the crude rac-1-{4-[2-hydroxy-3-(5-quinolyloxy)propyl]piperazin-1-yl}-2,2-diphenylethan-1-one fumarate of the formula V 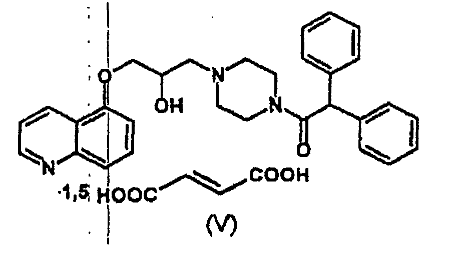 and subsequently
d) the crude rac-1-{4-[2-hydroxy-3-(5-quinolyloxy)propyl]piperazin-1-yl}-2,2-diphenylethan-1-one fumarate (V) thus formed is isolated and dissolved in a solvent mixture of methanol and methylene chloride, treated with activated carbon and subsequently filtered through a pressure filter having silica gel as column material, and the pure rac-1-{4-[2-hydroxy-3-(5-quinolyloxy)propyl]piperazin-1-yl}-2,2-diphenylethan-1-one fumarate (V) thus obtained is crystallized from a suitable alcohol.

2. Method according to Claim 1, **characterized in that** acetone is used as solvent.

3. Method according to Claim 1, **characterized in that** the reaction is carried out in a temperature range from 35 to 60°C.

4. Method according to Claim 1, **characterized in that** the amount of silica gel is 3 times the amount of starting material.

5. Method according to Claim 1, **characterized in that** the temperature during the crystallization in process stage (d) is 0°C.

6. Method according to one of Claims 1-5, **characterized in that** a rac-1-{4-[2-hydroxy-3-(5-quinolyloxy)propyllpiperazin-1-yl}-2,2-diphenylethan-1-one fumarate having a purity of greater than 98.54% is obtained.

7. Method according to one of Claims 1-5, **characterized in that** a rac-1-{4-[2-hydroxy-3-(5-quinolyloxy)propyllpiperazin-1-yl}-2,2-diphenylethan-1-one fumarate having a purity of at least 98.55% is obtained.

8. Method according to one of Claims 1-5, **characterized in that** a rac-1-{4-[2-hydroxy-3-(5-quinolyloxy)propyllpiperazin-1-yl}-2,2-diphenylethan-1-one fumarate having a purity of at least 99.55% is obtained.

9. Method according to one of Claims 1-5, **characterized in that** a rac-1-{4-[2-hydroxy-3-(5-quinolyloxy)propyl]piperazin-1-yl}-2,2-diphenylethan-1-one fumarate having a purity of at least 99.65% is obtained.

10. Method according to one of Claims 1-5, **characterized in that** a rac-1-{4-[2-hydroxy-3-(5-quinolyloxy)propyl]piperazin-1-yl}-2,2-diphenylethan-1-one fumarate having a purity of at least 99.84% is obtained.

11. Method according to one of Claims 1-5, **characterized in that** a rac-1-{4-[2-hydroxy-3-(5-quinolyloxy)propyl]piperazin-1-yl}-2,2-diphenylethan-1-one fumarate according to Claims 6 to 10 is obtained in an amount of greater than 1000 g.

12. Method according to one of Claims 1-5, **characterized in that** a rac-1-{4-[2-hydroxy-3-(5-quinolyloxy)propyl]piperazin-1-yl}-2,2-diphenylethan-1-one fumarate according to Claims 6 to 10 is obtained which, as impurity, comprises less than 0.1% of at least one of the individual impurities VU1-VU4 and VU6-VU11.

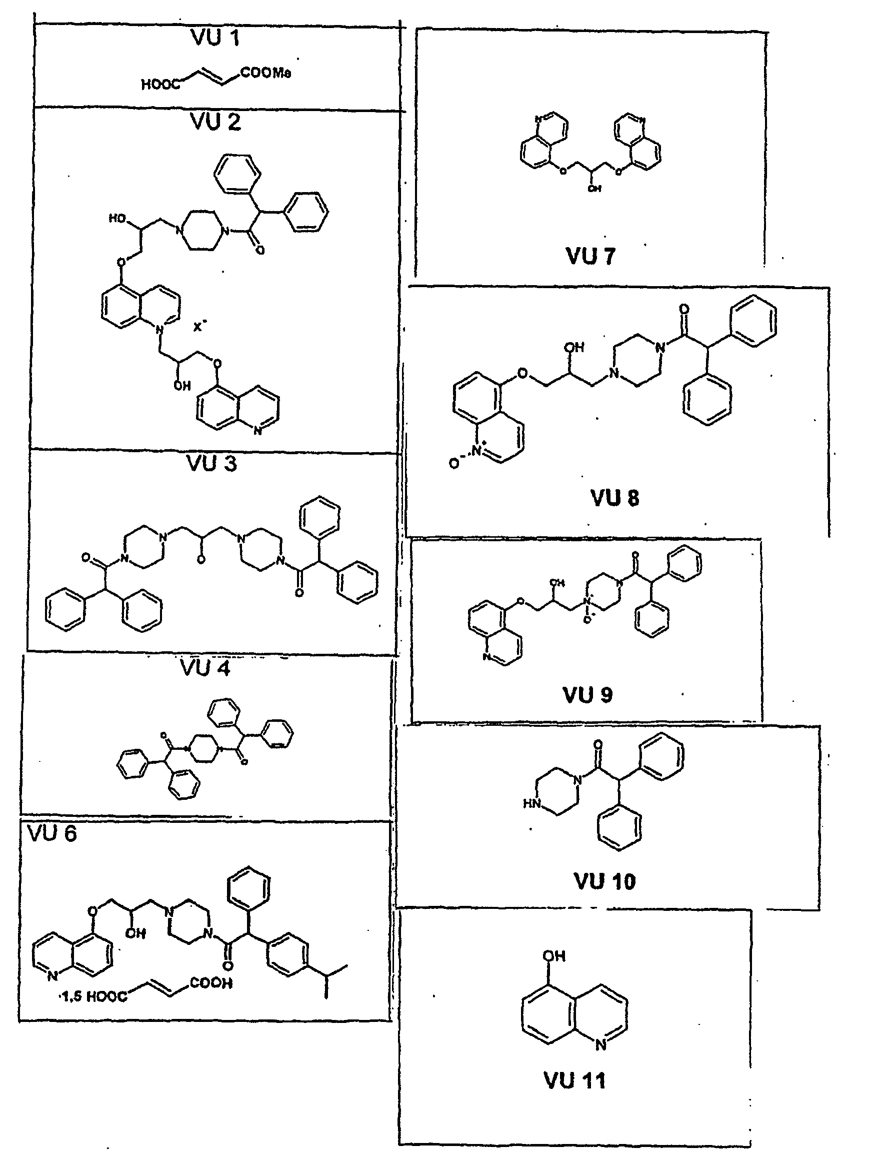

13. Method according to one of Claims 1-5, **characterized in that** a rac-1-{4-[2-hydroxy-3-(5-quinolyloxy)propyl]piperazin-1-yl}-2,2-diphenylethan-1-one fumarate according to Claims 6 to 10 is obtained which comprises, as impurity, less than 0.01% of at least one of the individual impurities VU1-VU4 and VU6-VU11.

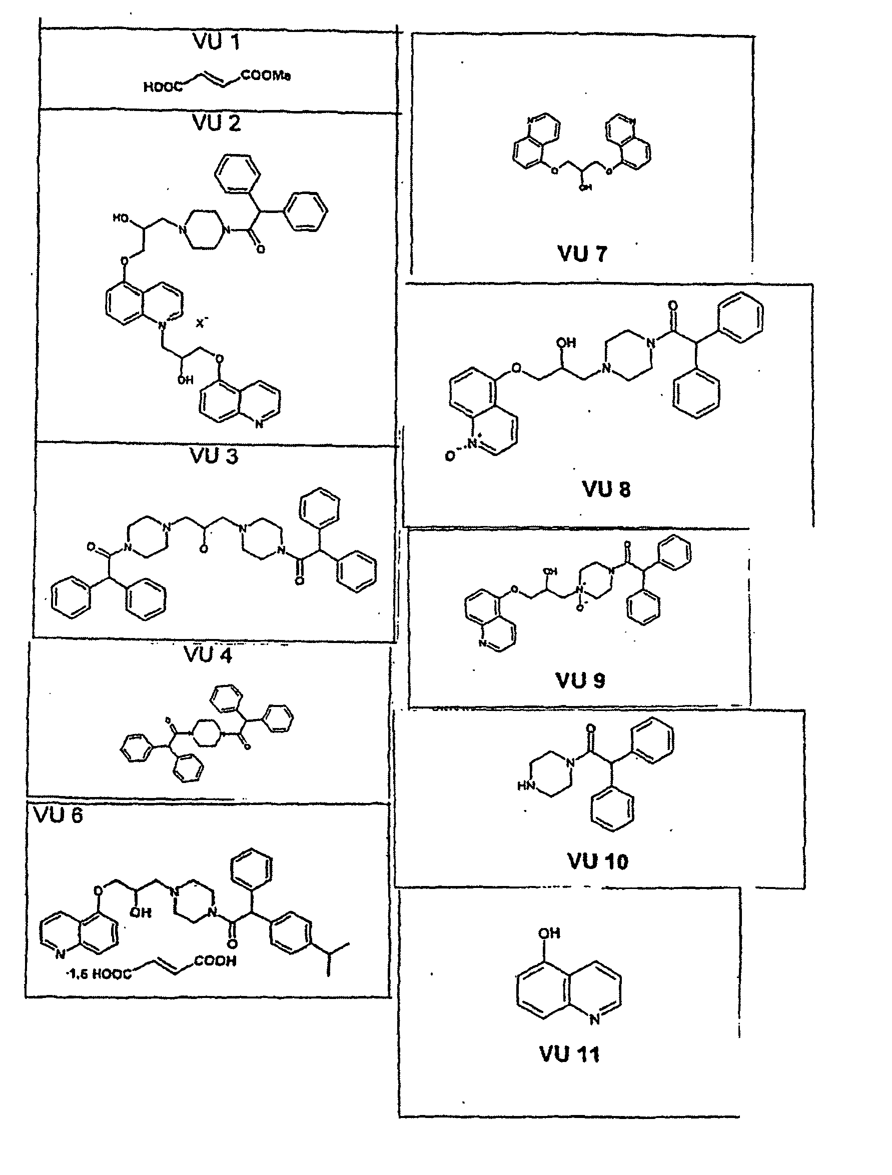

## Revendications

1. Procédé de préparation de fumarate de rac-1-{4-[2-hydroxy-3-(5-quinoléyloxy)propyl]-pipérazin-1-yl}-2,2-diphényléthan-1-one pur, **caractérisé en ce qu'**on fait d'abord réagir
a) un époxytosylate de structure I 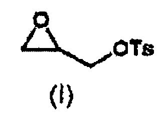 avec
b) de la 5-hydroxyquinoléine (II) 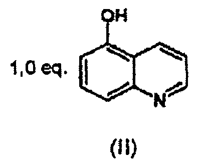 et du carbonate de césium dans un solvant approprié et à une température appropriée en 5-(2,3-époxypropoxy)-quinoléine de formule III 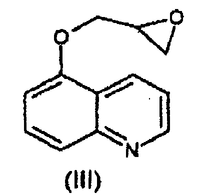 puis on transforme la 5-(2,3-époxypropoxy)-quinoléine de formule III
c) avec de la N-(2,2-diphénylacétyl)pipérazine de formule IV 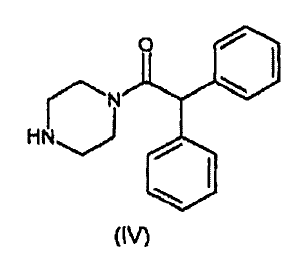 dans un solvant approprié et à une température appropriée, avec addition consécutive d'acide fumarique solide en fumarate de rac-1-{4-[2-hydroxy-3-(5-quinoléyloxy)propyl]-pipérazin-1-yl}-2,2-diphényléthan-1-one brut de formule V 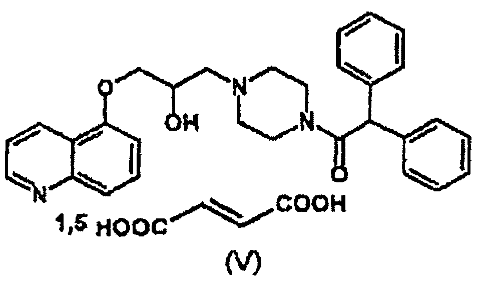 puis
d) le fumarate de rac-1-{4-[2-hydroxy-3-(5-quinoléyloxy)propyl]-pipérazin-1-yl}-2,2-diphényléthan-1-one (V) brut formé est isolé et dissous dans un mélange de solvants de méthanol et de chlorure de méthylène, traité avec du charbon actif et ensuite filtré via un filtre sous pression avec un gel silicique comme matériau de colonne et le fumarate de rac-1-{4-[2-hydroxy-3-(5-quinoléyloxy)propyl]-pipérazin-1-yl}-2,2-diphényléthan-1-one (V) pur ainsi obtenu est cristallisé à partir d'un alcool approprié.

2. Procédé selon la revendication 1, **caractérisé en ce qu'**on utilise de l'acétone comme solvant.

3. Procédé selon la revendication 1, **caractérisé en ce que** la transformation est réalisée dans une plage de température de 35 à 60°C.

4. Procédé selon la revendication 1, **caractérisé en ce que** la quantité de gel silicique représente la quantité triple du matériau de départ.

5. Procédé selon la revendication 1, **caractérisé en ce que** la température lors de la cristallisation dans l'étape de procédé (d) est de 0°C.

6. Procédé selon l'une quelconque des revendications 1-5, **caractérisé en ce qu'**on obtient du fumarate de rac-1-{4-[2-hydroxy-3-(5-quinoléyloxy)propyl]-pipérazin-1-yl}-2,2-diphényléthan-1-one présentant une pureté supérieure à 98,54%.

7. Procédé selon l'une quelconque des revendications 1-5, **caractérisé en ce qu'**on obtient du fumarate de rac-1-{4-[2-hydroxy-3-(5-quinoléyloxy)propyl]-pipérazin-1-yl}-2,2-diphényléthan-1-one présentant une pureté d'au moins 98,55%.

8. Procédé selon l'une quelconque des revendications 1-5, **caractérisé en ce qu'**on obtient du fumarate de rac-1-{4-[2-hydroxy-3-(5-quinoléyloxy)propyl]-pipérazin-1-yl}-2,2-diphényléthan-1-one présentant une pureté d'au moins 99,55%.

9. Procédé selon l'une quelconque des revendications 1-5, **caractérisé en ce qu'**on obtient du fumarate de rac-1-{4-[2-hydroxy-3-(5-quinoléyloxy)propyl]-pipérazin-1-yl}-2,2-diphényléthan-1-one présentant une pureté d'au moins 99,65%.

10. Procédé selon l'une quelconque des revendications 1-5, **caractérisé en ce qu'**on obtient du fumarate de rac-1-{4-[2-hydroxy-3-(5-quinoléyloxy)propyl]-pipérazin-1-yl}-2,2-diphényléthan-1-one présentant une pureté d'au moins 99,84%.

11. Procédé selon l'une quelconque des revendications 1-5, **caractérisé en ce qu'**on obtient du fumarate de rac-1-{4-[2-hydroxy-3-(5-quinoléyloxy)propyl]-pipérazin-1-yl}-2,2-diphényléthan-1-one selon les revendications 6 à 10 en une quantité supérieure à 1000 g.

12. Procédé selon l'une quelconque des revendications 1-5, **caractérisé en ce qu'**on obtient du fumarate de rac-1-{4-[2-hydroxy-3-(5-quinoléyloxy)-propyl]-pipérazin-1-yl}-2,2-diphényléthan-1-one selon les revendications 6 à 10, qui contient comme impureté moins de 0,1% d'au moins une des impuretés individuelles VU1-VU4 et VU6-VU11.

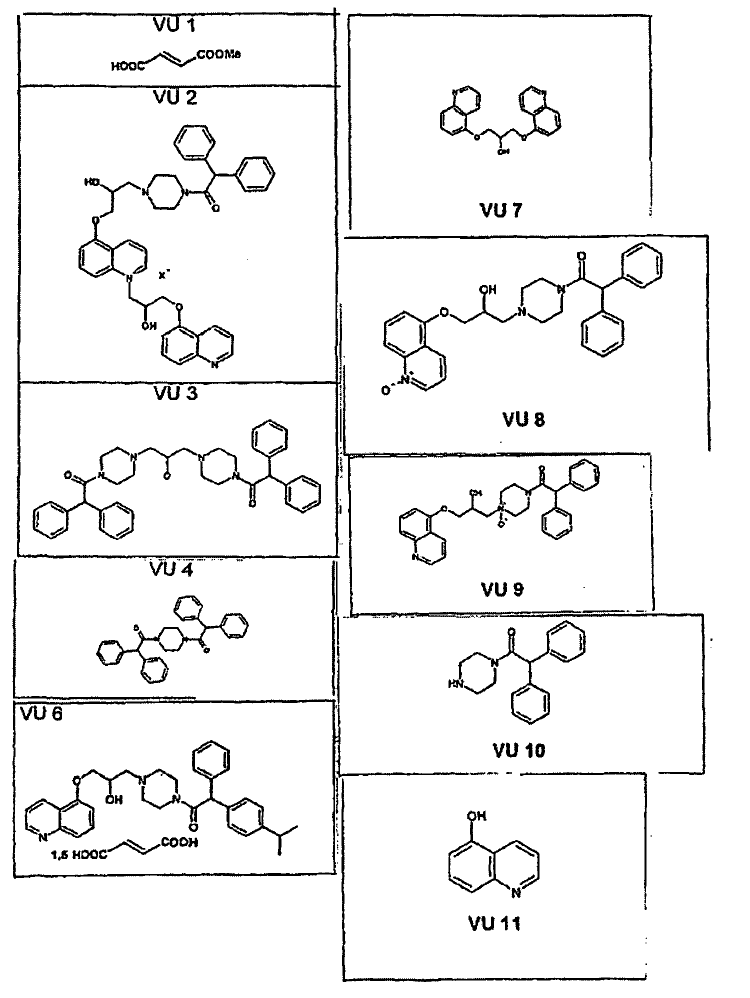

13. Procédé selon l'une quelconque des revendications 1-5, **caractérisé en ce qu'**on obtient du fumarate de rac-1-{4-[2-hydroxy-3-(5-quinoléyloxy)-propyl]-pipérazin-1-yl}-2,2-diphényléthan-1-one selon les revendications 6 à 10, qui contient comme impureté moins de 0,01% d'au moins une des impuretés individuelles VU1-VU4 et VU6-VU11.
